# EUROPEAN PATENT APPLICATION

(11) **EP 1 232 735 A2**
(43) Date of publication of application: **21.08.2002**
(21) Application number: 02003709.9
(22) Date of filing: 19.02.2002
(51) Int. Cl.: A61F 13/15, A61F 13/49, A61F 13/511

(54) **Diaper cover and method for manufacturing a diaper cover**

(30) Priority: 20.02.2001 SE 0100575; 20.02.2001 US 269380 P
(71) Applicant: SCA Hygiene Products AB, 405 03 Göteborg (SE); TYTEX A/S, 7430 Ikast (DK)
(72) Inventor: Carlbark, Olle, 41757 Göteborg (SE); Rönnberg, Peter, 43133 Mölndal (SE); Strannemalm, Kenneth, 44831 Floda (SE); Balslev Sörensen, Bettina, 8600 Silkeborg (DK)
(74) Representative: Hammond, Andrew David

(57) **Abstract**

A diaper cover (10) having a layered structure (22), the layered structure including an inwardly facing layer (24) facing towards a wearer and an outwardly facing layer (26) facing away from the wearer. The layered structure also has at least one line of stitching (32; 36; 44). The stitching is at least partially formed by coloured thread to thereby form a line of coloured stitching. The coloured thread has a colour contrasting to material of the diaper cover (10) adjacent the line of coloured stitching, the colour of the coloured thread being indicative of the predetermined size of the diaper cover.

## Description

### TECHNICAL FIELD:

The present invention relates to a diaper cover according to the preamble of claim 1. The invention further relates to a method of manufacturing a diaper cover.

### BACKGROUND OF THE INVENTION:

Washable reusable diaper covers are used primarily when caring for elderly and/or incontinent adults. Such diaper covers are intended to be used with disposable absorbent inserts which are held in place against the crotch region of the body of the wearer by the diaper cover. One example of a diaper cover is described in JP-A-8131469.

Wearers of diaper covers vary considerably in size. To reduce the risk of leakage and to enhance comfort and feeling of security, it is important that the diaper cover fits snugly to the body of the wearer. Accordingly, it is necessary that several sizes of diaper cover be provided. Particularly in institutes caring for many afflicted residents, it is advantageous if the nursing staff can easily distinguish between the various sizes of diaper cover to thereby quickly and confidently select the correct size of diaper cover required by each particular resident.

The present applicant has been marketing a range of diaper covers under the trade name Tena Cover in which the various sizes of the diaper covers are identifiable by colour coding of braiding around at least a portion of the periphery of the diaper cover. Thus, for example, a diaper cover of "medium" size may be provided with blue braiding. Although the colour coding of braiding allows the various sizes of diaper covers to be quickly and reliably determined, the manufacturing of the diaper covers is complicated by the fact that stocks of different coloured strips of braiding must be provided.

### SUMMARY OF THE INVENTION:

It is therefore an object of the present invention to provide a diaper cover, the size of which can be readily determined, which diaper cover can be manufactured in a more rational manner than prior diaper covers.

This object is achieved in accordance with the present invention by a diaper cover as claimed in claim 1 and a method of manufacturing according to claim 10.

Since, in accordance with the present invention, the size of the diaper cover is indicated by the colour of a line of coloured stitching, and said line of coloured stitching can easily be incorporated at any suitable location on the diaper cover, the need to produce, stock and manage different coloured braiding is obviated. Rather, all that is needed are reels of suitably coloured thread.

Advantageous embodiments of the diaper cover and method of manufacture according to the present invention are detailed in the dependent claims.

### BRIEF DESCRIPTION OF THE DRAWINGS:

The invention will be described in greater detail in the following by way of example only and with reference to embodiments shown in the attached drawings, in which:
Fig. 1 is a schematic plan view of a diaper cover according to the present invention, and
Fig. 2 is a schematic sectional view taken along line II-II of Fig. 1, though on a larger scale.

### DETAILED DESCRIPTION OF PREFERRED EMBODIMENTS:

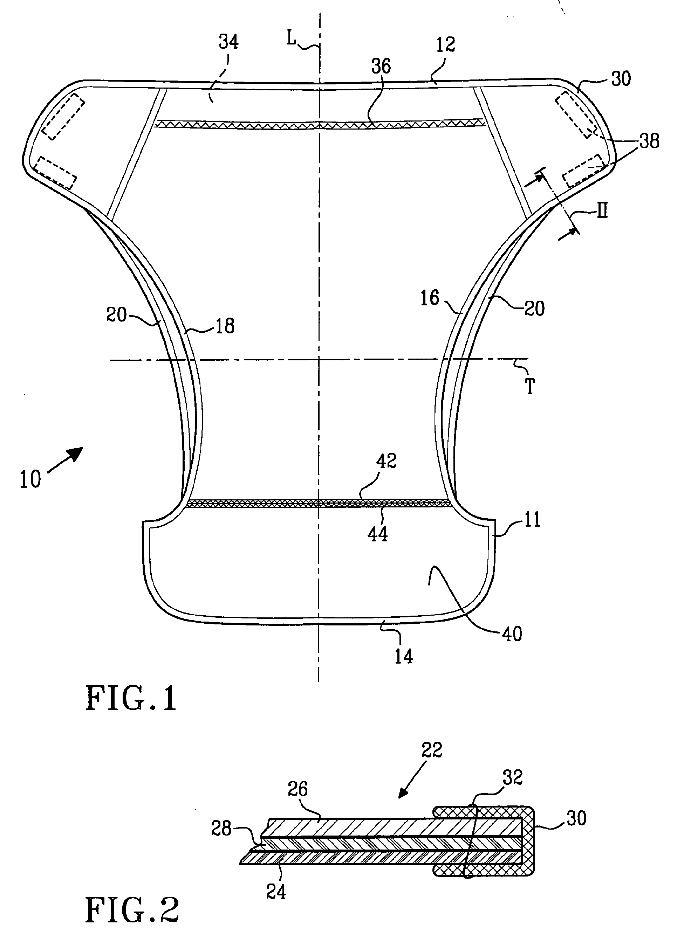

In Fig. 1, reference numeral 10 generally denotes a diaper cover in accordance with the present invention. The expression "diaper cover" is intended to encompass any reusable garment which is worn by a wearer with the express purpose of assisting in maintaining an absorbent insert in proximity to the crotch region of the wearer. The expression "reusable garment" is intended to imply that the diaper cover may be laundered and repeatedly used, in contrast to a disposable garment which is intended to be discarded after having been subjected to an insult of bodily discharges.

The diaper cover of the present invention has a longitudinal extension about a longitudinal axis L and a transverse extension about a transverse axis T perpendicular to the longitudinal axis. The cover further exhibits a periphery 11 comprising a first end edge 12 extending substantially parallel to the transverse axis T, and a second end edge 14 remote from the first end edge, the second end edge also extending substantially parallel to the transverse axis. The periphery 11 also includes opposed longitudinal edges 16, 18 respectively extending between the first and second end edges and forming leg openings when the diaper cover is fastened around the waist of the wearer. In a manner which is known per se, the diaper cover may be provided with so-called standing gathers 20 adjacent the longitudinal edges 16, 18 to assist in retaining bodily discharges within the diaper cover.

In Fig. 1, the diaper cover 10 is illustrated with its surface which would normally be directed away from a wearer facing upwardly. As is most readily apparent from Fig. 2, the diaper cover comprises a layered structure, generally denoted by 22. The layered structure comprises an inwardly facing layer 24 facing towards a wearer and an outwardly facing layer 26 facing away from the wearer. In the illustrated embodiment, the layered structure 22 further comprises a third layer 28 positioned between the inwardly facing layer 24 and the outwardly facing layer 26. The various materials of the layered structure 22 should be selected so that the diaper cover 10 exhibits acceptable softness, strength and durability, even after being subjected to a plurality of wash cycles. Advantageously, the layered structure 22 is formed from a laminate, wherein the inwardly facing layer 24 and the outwardly facing layer 26 are of the same material, while the third layer 28 is of different material. Preferably, the material of the inwardly and outwardly facing layers is a polyester fabric having a basis weight of about 120 g/m². In order to ensure that the diaper cover will assist in retaining any bodily fluids which may leak from the absorbent insert though, for reasons of comfort, still be breathable, the third layer is preferably a microporous hydrophilic polyurethane membrane having a basis weight of about 30 g/m². The layers of the layered structure may be laminated together using a polyurethane hot-melt adhesive.

For increased comfort and durability, the periphery 11 of the diaper cover may be provided with braiding 30. The braiding 30 may be in the form of a strip of material of 15 - 20 mm width which, as clearly shown in Fig. 2, is folded around the edges of the layered structure 22. Preferably, the braiding is a cotton/polyester material containing elastic threads to impart elasticity to the braiding. The braiding 30 may be stitched to the layered structure in a conventional manner using one or more lines of stitching 32.

To improve fit and comfort, the diaper cover may be provided with an elasticated waist band 34. The waist band 34 may be positioned adjacent and along the first end edge 12 on the inwardly facing layer 24 of the layered structure. The waist band is advantageously of the same material as the braiding 30, though of somewhat greater width, for example 22 mm. The waist band is attached to the layered structure in a stretched condition preferably by stitching, utilizing the line of stitching 32 which secures the braiding to the layered structure as well as a further line or lines of stitching 36 running substantially parallel to the first end edge 12.

To allow the diaper cover 10 to be fastened about the waist of a wearer, a suitable fastener system is employed. Since the diaper cover is to be laundered, a mechanical fastener system is preferred over an adhesive system. Conceivable mechanical fastener systems include complementary buttons and button holes, studs and fasteners, and hook-and-loop systems. In the preferred illustrated embodiment, the fastener system is a hook-and-loop system comprising patches 38, 40 of complementary fastener material. Thus, in the illustrated embodiment, the diaper cover 10 is provided on its inwardly facing layer 24 with patches 38 of hook material in the vicinity of the first end edge 12. Although the patches 38 of hook material have been illustrated as being attached to the inwardly facing layer 24, it is to be understood that the present invention also encompasses embodiments in which patches of hook material are in the form of tabs extending outwardly from the longitudinal edges 20 of the diaper cover. Preferably, the patches 38 of hook material are stitched to the layered structure 22 by a peripheral line or lines of stitching (not shown).

The cover is further provided on its outwardly facing layer 26 with a patch of loop material in the form of a panel 40 extending adjacent and along the second end edge 14 for the entire transverse extension of the diaper cover. The panel 40 of loop material extends in the longitudinal direction from the second end edge 14 towards the first end edge 12 and terminates with a transverse edge 42 remote from the end edges. To ensure an adequate area of loop material to which the patches 38 of hook material can be fastened, the panel 40 of loop material should extend between about one quarter and one third the length in the longitudinal direction of the diaper cover. The panel of loop material may be made from any suitable material, such as a polyamide having a basis weight of about 200 g/m². In a manner similar to the waist band, the panel 40 of loop material is attached to the layered structure preferably by stitching, utilizing the line of stitching 32 which secures the braiding to the layered structure as well as a further line or lines of stitching 44 running substantially parallel to the transverse axis T.

It is proposed that the diaper cover 10 in accordance with the present invention be manufactured in a plurality of different sizes. In order to easily distinguish between the various sizes, and in accordance with the present invention, at least one line of stitching on the diaper cover is at least partially formed by coloured thread to thereby form a line of coloured stitching. The coloured thread must have a colour contrasting to material of the diaper cover adjacent the line of coloured stitching, with the colour of the coloured thread being indicative of the predetermined size of the diaper cover. The expression "having a colour contrasting to material of the diaper cover" means that the line of coloured stitching must be visible to the naked eye in broad daylight by a person with normal vision (i.e. not colour-blind, nor long or short sighted) at a distance of 2 metres from the diaper cover. Purely by way of example, the diaper cover may be made in three different sizes, namely "small", "medium" and "large". Yellow thread could then be used to denote a small sized diaper cover, blue thread could be used to denote a medium sized diaper cover and brown thread could be used to denote a large sized diaper cover.

The line of coloured stitching may be located anywhere on the diaper cover. Preferably, the line of coloured stitching extends spaced from the first and second end edges 12, 14 substantially parallel to the transverse axis T. The type of stitching used may be selected such that the line of coloured stitching is visible on the outwardly facing layer 26, the inwardly facing layer 24 or both. To rationalize production, it is advantageous if the line of coloured stitching serves to at least partially join together components of the diaper cover. Thus, the line of stitching 36 which runs substantially parallel to the first end edge 12 and is used to secure the waist band 34 to the layered structure 22 may advantageously be formed by coloured thread. This applies also to the line or lines of stitching 44 used to at least partially secure the panel 40 of loop material to the layered structure. It is also conceivable that a line of coloured stitching be used to secure the patches 38 of hook material and/or the braiding 30 to the layered structure 22. In such cases, the patches and the braiding itself must of course be of a contrasting colour to the thread. Alternatively, the braiding 30 may be stitched to the layered structure or diaper cover using thread different to the coloured thread.

The line of coloured stitching need not necessarily be a single line. It falls within the scope of the present invention to employ any suitable stitching pattern, for example a pattern having a lateral extension of several millimeters.

The diaper cover 10 of the present invention may advantageously be manufactured by forming the layered structure 22 by at least indirectly joining the inwardly facing layer 24 and the outwardly facing layer 26, providing a line of coloured stitching on the layered structure 22, and attaching braiding 30 around the periphery 11 of the diaper cover.

It is to be understood that the invention has been described above and illustrated in the drawings purely by way of example. Accordingly, the skilled person will appreciate that the present invention may be varied within the scope of the appended claims. For example, the relative positions of the patches of hook and loop material may be interchanged.

## Claims

1. A diaper cover (10) of predetermined size, said diaper cover having:
a longitudinal extension about a longitudinal axis (L);
a transverse extension about a transverse axis (T) perpendicular to said longitudinal axis;
a first end edge (12) extending substantially parallel to said transverse axis (T), and
a second end edge (14) remote from said first end edge (12), said second end edge extending substantially parallel to said transverse axis (T);
said diaper cover further having a layered structure (22), said layered structure comprising an inwardly facing layer (24) facing towards a wearer and an outwardly facing layer (26) facing away from the wearer, said layered structure further comprising at least one line of stitching (32; 36; 44),
**characterized in that** at least one of said at least one line of stitching is at least partially formed by coloured thread to thereby form a line of coloured stitching, said coloured thread having a colour contrasting to material of said diaper cover (10) adjacent said line of coloured stitching, said colour of said coloured thread being indicative of said predetermined size of said diaper cover.

2. The diaper cover (10) as claimed in claim 1, **characterized in that** said line of coloured stitching (32; 36; 44) extends spaced from said first and second end edges (12, 14) substantially parallel to said transverse axis (T).

3. The diaper cover (10) as claimed in claim 1 or 2, **characterized in that** said line of coloured stitching (32; 36; 44) is visible on said outwardly facing layer (26).

4. The diaper cover (10) as claimed in claim 1 or 2, **characterized in that** said line of coloured stitching (32; 36; 44) is visible on said inwardly facing layer (24).

5. The diaper cover (10) as claimed in claim 1 or 2, **characterized in that** said line of coloured stitching (32; 36; 44) is visible on both said outwardly facing layer (24) and said inwardly facing layer (24).

6. The diaper cover (10) as claimed in any one of claims 2 to 5, **characterized in that** said line of coloured stitching (32; 36; 44) serves to at least partially join together components of the diaper cover.

7. The diaper cover (10) as claimed in claim 6, **characterized in that** said components include at least one patch (38; 40) of fastener material comprised in a fastener system to fasten the diaper cover about the wearer.

8. The diaper cover (10) as claimed in claim 7, **characterized in that** said at least one patch of fastener material is a panel (40) of loop material extending from said second end edge (14) towards said first end edge (12), said panel (40) of loop material terminating in a transverse edge (42) remote from said second end edge, with said line of coloured stitching (44) extending adjacent to and along said transverse edge (42).

9. The diaper cover (10) as claimed in claim 6, 7 or 8, **characterized in that** said components include a strip of waist elastic (34) adjacent said first end edge (12).

10. A method of manufacturing a diaper cover (10) of predetermined size, said diaper cover having:
a longitudinal extension about a longitudinal axis (L);
a transverse extension about a transverse axis (T) perpendicular to said longitudinal axis; and
a periphery (11), said periphery comprising
a first end edge (12) extending substantially parallel to said transverse axis, and
a second end edge (14) remote from said first end edge, said second end
edge extending substantially parallel to said transverse axis; said diaper cover (10) further having a layered structure (22), said layered structure comprising an inwardly facing layer (24) facing towards a wearer and an outwardly facing layer (26) facing away from the wearer, said layered structure further comprising at least one line of stitching (32; 36; 44), with at least one of said at least one line of stitching being at least partially formed by coloured thread to thereby form a line of coloured stitching, said coloured thread having a colour contrasting to material of said diaper cover adjacent said line of coloured stitching, said colour of said coloured thread being indicative of said predetermined size of said diaper cover; said method comprising:
forming said layered structure (22) by at least indirectly joining said inwardly facing layer (24) and said outwardly facing layer (26);
providing said line of coloured stitching (32; 36; 44) on said layered structure, and
attaching braiding (30) around said periphery (11) of said diaper cover.

11. The method as claimed in claim 10, whereby the step of attaching braiding comprises attaching said braiding (30) by stitching using a thread different to said coloured thread.

12. The method as claimed in claim 10 or 11, whereby the step of providing said line of coloured stitching (32; 36; 44) comprises at least partially joining together components of the diaper cover by said line of coloured stitching.

13. The method as claimed in claim 12, whereby said line of coloured stitching (44) at least partially joins a patch of fastener material (38; 40) to said diaper cover.

14. The method as claimed in either claim 12 or 13, whereby said line of coloured stitching (36) at least partially joins a strip of waist elastic to said diaper cover.
